# FASCICULE DE BREVET EUROPEEN

(11) **EP 0 370 841 B1**
(45) Date de publication et mention de la délivrance du brevet: **09.06.1993**
(21) Numéro de dépôt: 89402829.9
(22) Date de dépôt: 13.10.1989
(51) Int. Cl.: G10K 11/00, G01S 15/89, A61F 7/00

(54) **Dispositif de traitement par ultrasons utilisant une céramique piézoélectrique focalisante et oscillante**
Ultraschallbehandlungsgerät, gebrauchmachend von einer fokalisierenden und oszillierenden piezoelektrischen Keramikvorrichtung
Ultrasound treatment apparatus using a focalizing and oscillating piezoelectric ceramic device

(30) Priorité: 27.10.1988 FR 8814015
(43) Date de publication de la demande: 30.05.1990
(73) Titulaire: EDAP INTERNATIONAL, F-77200 Croissy Beaubourg-Marne la Vallée (FR)
(72) Inventeur: Dory, Jacques, F-77450 Coupvray (FR)
(74) Mandataire: Hirsch, Marc-Roger

(56) Documents cités:
- EP-A- 0 170 416
- EP-A- 0 194 897
- DE-A- 3 618 082
- DE-A- 3 736 733
- PROCEEDINGS OF THE IEEE 1986 ULTRASONICS SYMPOSIUM, Williamsburg, VA, 17-19 novembre 1986, vol. 2, IEEE, New York, US; F.L. LIZZI et al.: "A therapeutic ultrasound system incorporating real-time ultrasonic scanning"

## Description

L'invention se rapporte au traitement par un faisceau focalisé d'ultrasons d'anomalies anatomiques et, plus particulièrement, de structures peu profondes pour lesquelles la tache focale du faisceau doit être relativement fine.

Le brevet français n° 84 06877 décrit un dispositif d'hyperthermie utilisant un transducteur de puissance en forme de coupelle sphérique qui engendre des trains d'ondes ultrasonores ayant par exemple des fréquences de l'ordre de 500 KHz. La localisation de la cible anatomique et son observation pendant les tirs s'effectuent au moyen d'un transducteur auxiliaire mécaniquement couplé à la coupelle et excité par un générateur d'impulsions échographiques ayant par exemple une fréquence de 5 MHz.

Ce dispositif est apte à traiter des tissus profonds, les ondes à la fréquence de 500 KHz subissant une absorption relativement faible au cours de leur propagation dans les tissus. Compte tenu de sa taille (diamètre de 200 à 300 mm par exemple) la coupelle n'est pas susceptible d'être soumise à un mouvement rapide qui permettrait un balayage échographique en temps réel et, en tout état de cause, sa fréquence de fonctionnement ne serait pas adaptée à la formation d'images échographiques de bonne qualité.

Il a été proposé notamment dans la demande de brevet japonais publiée N° 58 188431 d'utiliser alternativement le transducteur de puissance pour le traitement et pour l'échographie. Lorsqu'on met en oeuvre un tel principe avec un transducteur de puissance fixe, ou bien on effectue une échographie de type A qui ne fournit que des indications de distance de la cible, ou bien on engendre un balayage du faisceau échographique en excitant le transducteur avec des ondes déphasées entre elles. Cette dernière solution est compliquée et ne fournit pas un balayage de la cible dans un angle suffisant. En outre, ce principe ne permet pas l'observation de la cible pendant la séquence de traitement.

DE- A-37 36 733 décrit un dispositif dans lequel le transducteur de puissance est utilisé pour l'échographie et oscille au cours d'une première phase d'utilisation qui permet de s'assurer que le faisceau émis ne rencontre pas de bulle d'air dans les tissus à traiter. Cette phase permet de régler la visée et est suivie d'une phase de traitement pendant laquelle l'échographie est arrêtée et le transducteur est immobile.

Ce dispositif ne permet évidemment pas d'effectuer une échographie dans un large champ d'observation autour de la cible pendant que celle-ci est traitée.

L'invention propose de faire osciller périodiquement le transducteur de puissance autour d'une position donnée pendant la durée d'utilisation, avec une vitesse et une amplitude d'oscillation propres à l'obtention d'un balayage échographique en temps réel dans un espace de tir donné, de capter la position du transducteur et de l'exciter avec des ondes de traitement à des moments où il oscille dans une région restreinte prédéterminée de son domaine d'oscillation, et de l'exciter avec des ondes échographiques pendant les intervalles entre lesdits moments.

Ainsi, compte tenu de la durée relativement faible de chaque intervalle de tir par rapport à la période d'oscillation, la puissance moyenne sera suffisante dans certains traitements.

L'invention s'applique principalement au traitement de structures situées à quelques cm seulement sous la peau, par exemple des structures oculaires ou des vaisseaux sanguins. Dans de telles applications, on utilise généralement des ondes de traitement à des fréquences de l'ordre de 5 à 10 MHz, qui procurent les taches focales très fines nécessaires. Bien que l'absorption de ces ondes dans les tissus soit très forte, la puissance moyenne requise reste de l'ordre de 1 KW, à cause de la faible distance de propagation et du fait que l'échauffement des tissus est important.

Le transducteur de puissance pouvant alors avoir un diamètre relativement faible, par exemple de l'ordre de 70 mm, son entraînement en oscillation est réalisable au moyen d'un moteur électrique.

D'autres particularités, ainsi que les avantages de l'invention, apparaîtront clairement à la lumière de la description ci-après.

Aux dessins annexés :
La figure 1 représente schématiquement un dispositif de traitement conforme à un mode d'exécution préféré de l'invention ;
La figure 2 illustre les formes d'ondes correspondantes, et
La figure 3 montre l'ensemble transducteur.

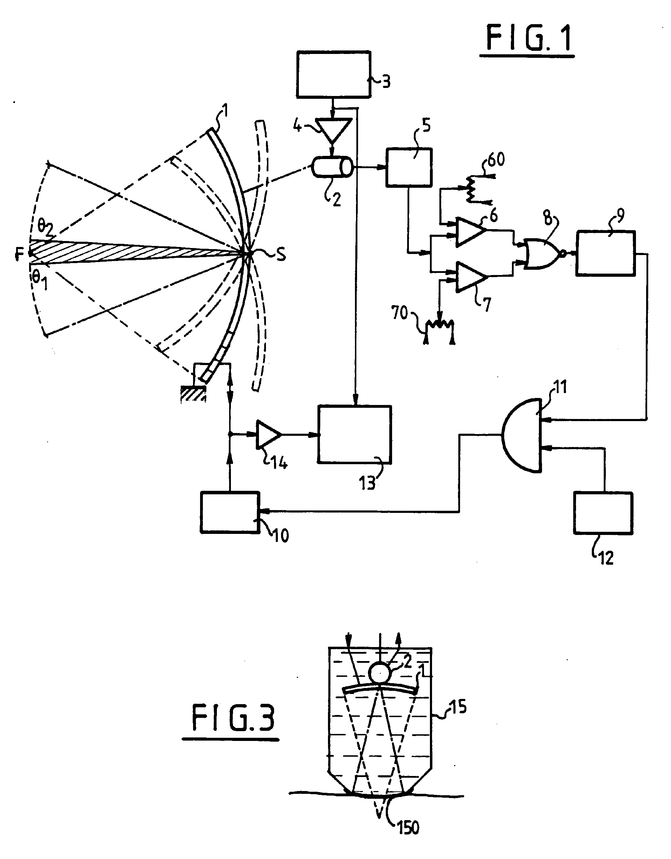

A la figure 1, on a représenté une coupelle sphérique en céramique piézoélectrique 1, montée oscillante de préférence autour d'un axe tangent à la coupelle au sommet S de celle-ci. L'amplitude angulaire d'oscillation sera, à titre d'exemple, comprise entre 30 et 60°. On a représenté en trait mixte les limites du secteur plan ainsi balayé par l'axe SF de la coupelle et en tirets le faisceau focalisé en F, émis par la coupelle dans sa position médiane.

Le mouvement oscillant de la coupelle est, de façon connue en soi, obtenu au moyen d'un moteur électrique 2 commandé par un générateur de balayage 3 par l'intermédiaire d'un amplificateur 4. Un capteur de position angulaire 5, de type connu en soi, fournit un signal électrique d'amplitude variable en fonction linéaire de l'angle ϑ que fait l'axe SF avec sa position médiane de référence.

A titre d'exemple, un tel capteur peut comporter un aimant permanent solidaire de l'arbre du moteur et coopérant avec un capteur de champ magnétique.

Le signal indicatif de ϑ est appliqué à deux amplificateurs opérationnels 6 et 7 qui reçoivent, par ailleurs, deux signaux de référence indicatifs de deux valeurs particulières de ϑ, soient ϑ₁ et ϑ₂. Ces signaux de référence sont réglables au moyen de potentiomètres, 60 et 70 respectivement.

Les sorties des amplificateurs 6 et 7, qui sont respectivement au niveau 1 lorsque ϑ≧ϑ₁ et ϑ≧ϑ₂, sont reliées aux entrées d'une porte OU EXCLUSIF 8 qui commande un modulateur 9. La sortie du modulateur 9 est reliée à l'entrée de commande d'un émetteur 10 par l'intermédiaire d'une porte OU 11, laquelle reçoit, par ailleurs, des signaux de synchronisation fournis par une horloge 12. L'émetteur 10 attaque la coupelle 1.

Celle-ci a par exemple un diamètre de 70 mm et est composée, de façon connue en soi, d'une pluralité d'éléments piézoélectriques isolés les uns des autres et juxtaposés pour constituer une mosaïque.

L'émetteur 10 peut en réalité comporter plusieurs dispositifs d'émission excitant chacun un groupe d'éléments de la coupelle. Cette technique étant connue, en considèrera, pour la clarté de l'exposé, que l'émetteur 10 n'engendre qu'un seul signal comportant, comme on l'expliquera dans la suite, à la fois des trains d'ondes de puissance destinés au traitement et des impulsions échographiques. Pour ces deux typos d'émission, la fréquence porteuse sera avantageusement la même, par exemple 5 MHz.

La coupelle sert à la fois de transducteur d'émission des ondes de puissance et des impulsions échographiques, et de transducteur de réception des échos formés par la réflexion des impulsions échographiques sur la cible.

Les échos reçus sont transmis à un dispositif échographique 13, de type connu en soi, par l'intermédiaire d'un amplificateur 14.

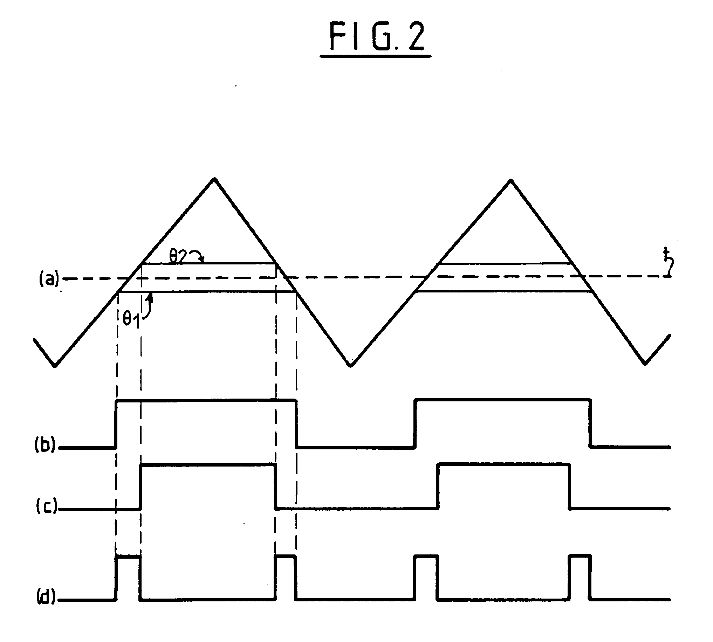

La figure 2 montre en (a) le signal en dents de scie engendré par le générateur de balayage 3. A titre d'exemple, ce signal a une période de 1/5 sec., si bien que la fréquence d'oscillation de la coupelle est de 5 Hz.

On a représenté en (b) le signal de sortie de l'amplificateur 6, lequel est au niveau 1 lorsque ϑ≧ϑ₁, et en (c), le signal de sortie de l'amplificateur 7, lequel est au niveau 1 lorsque ϑ≧ϑ₂. Le signal (d) à la sortie de la porte 8 comporte ainsi un créneau au niveau logique 1 lorsque ϑ ₁≦ϑ≦ϑ₂, c'est-à-dire deux fois par période. Dans les intervalles entre ces créneaux (d), la porte 11 transmet à l'émetteur 10 les impulsions de l'horloge 12, qui ont par exemple une haute fréquence de 5 MHz (donc une durée de 0,2 µs) et une fréquence de récurrence de 10 KHz. Ces valeurs sont appropriées à la formation d'une image échographique de bonne qualité avec une fréquence de balayage de 5 Hz. Pendant la durée de l'émission échographique il peut être envisagé de faire travailler l'émetteur 10 à puissance réduite, par exemple, en réduisant sa tension d'alimentation.

Pendant la durée de chaque créneau (d ) qui sera par exemple de l'ordre de 20ms, l'émetteur 10 engendre des ondes de traitement à la haute fréquence de 5 MHz.

Les valeurs de ϑ₁ et ϑ₂ sont réglées pour que la cible soit située dans l'angle de tir.

Après une localisation initiale de la cible effectuée en déplaçant dans l'espace la coupelle oscillante jusqu'à amener en coïncidence un repère matérialisant, sur l'écran du dispositif échographique, la position théorique de son foyer F, avec l'image échographique de la zone à traiter, obtenue comme on vient de l'indiquer, on peut régler ϑ₁, et ϑ₂ pour viser une cible précise dans la zone à traiter. En cas de déplacement de la cible au cours du traitement ou si l'on constate que les tirs sont mal ciblés, il suffit de réajuster la localisation par un petit déplacement de la coupelle en oscillation.

La figure 3 montre que la coupelle oscillante 1 et le moteur 2 qui l'entraîne en oscillation sont immergés dans un liquide de couplage contenu dans un boîtier 15 dont la face avant est munie d'une membrane déformable 150 transparente aux ultrasons. Cette membrane a une surface suffisante pour transmettre le cône de balayage échographique et est amenée au contact de la peau du patient.

En fonction de la profondeur de la tumeur, les ondes de traitement seront émises à l'intérieur de chaque créneau par trains discontinus, la durée d'émission pour chaque train pouvant varier entre quelques dizaines et quelques centaines de µs. La puissance de crête d'émission, d'autant plus grande que le train est plus bref, pourra atteindre plusieurs centaines de KW, les puissances de crête élevées et les émissions brèves ayant l'avantage d'éviter la diffusion thermique de l'énergie qui réduirait la précision des tirs.

Lorsque la puissance moyenne souhaitée pour le traitement est élevée, il peut être envisagé de faire varier la vitesse du moteur au cours de chaque cycle de balayage de façon à ralentir le balayage pendant les créneaux d'émission des ondes de traitement.

A la place du balayage sectoriel, on pourra envisager d'utiliser un balayage linéaire et, à cet effet, de communiquer au transducteur un mouvement linéaire d'oscillation.

## Revendications

1. Dispositif de traitement par ultrasons comportant un transducteur de puissance en forme de coupelle autofocalisante (1) associé à un générateur d'excitation de puissance (10) et à un dispositif d'échographie (13) utilisant ledit transducteur comme récepteur d'échos,
caractérisé par des moyens (2) de faire osciller périodiquement ledit transducteur autour d'une position donnée, pendant la durée d'utilisation, avec une vitesse et une amplitude d'oscillation propres à l'obtention d'un balayage échographique en temps réel dans un espace de tir donné, et par des moyens (5 à 12) de capter la position du transducteur et d'exciter ledit transducteur avec des ondes de traitement à des moments où le transducteur oscille dans une région restreinte prédéterminée de son domaine d'oscillation, et de l'exciter avec des ondes échographiques pendant les intervalles entre lesdits moments.

2. Dispositif selon la revendication 1,
caractérisé en ce que le balayage est un balayage sectoriel du type B et lesdits moments correspondent au passage du faisceau dans un secteur angulaire restreint prédéterminé.

3. Dispositif selon la revendication 2,
caractérisé en ce que ledit secteur restreint est défini par un capteur de position angulaire (5) tandis que les moments d'émission de l'onde de traitement sont définis, à partir des indications dudit capteur, par des moyens logiques (6 à 11) générateurs de créneaux d'émission de puissance.

4. Dispositif selon la revendication 3,
caractérisé en ce que l'émission échographique et l'émission de puissance utilisent un même générateur d'excitation (10) travaillant à la même fréquence.

5. Dispositif selon la revendication 1,
caractérisé par un mouvement linéaire d'oscillation du transducteur.

## Patentansprüche

1. Ultraschall-Verarbeitungsvorrichtung mit einem Leistungswandler in Form einer autofokussierenden Schale (1), der einem Leistungsanregungsgenerator (10) und einer Echographievorrichtung (13) zugeordnet ist, die den Wandler als Echoempfänger verwendet, gekennzeichnet durch Mittel (2), um diesen Wandler währen der Betriebsdauer mit einer Geschwindigkeit und einer Amplitude, die für ein echographisches Abtasten eines gegebenen Schußraums in Echtzeit geeignet sind, periodisch um eine gegebene Stellung schwingen zu lassen, und durch Mittel (5 bis 12), um die Stellung des Wandlers zu erfassen und diesen Wandler mit Verarbeitungswellen zu Zeitpunkten anzuregen, in denen der Wandler in einer begrenzten vorbestimmten Zone seines Oszillationsbereichs schwingt, und ihn mit echographischen Wellen während des Intervalle zwischen diesen Zeitpunkten anzuregen.

2. Vorrichtung nach Anspruch 1, dadurch gekennzeichnet, daß die Abtastung ein sektorielles Überstreichen vom Typ B ist und daß die Zeitpunkte dem Durchgang des Strahls durch einen begrenzten vorbestimmten Winkelsektor entsprechen.

3. Vorrichtung nach Anspruch 2, dadurch gekennzeichnet, daß der begrenzte Sektor definiert wird durch einen Winkelstellungsgeber (5), während die Sendezeitpunkte der Verarbeitungswelle ausgehend von Angaben des Gebers durch logische Mittel (6 bis 11) definiert werden, die Rechtecksignale zur Leistungsaussendung erzeugen.

4. Vorrichtung nach Anspruch 3, dadurch gekennzeichnet, daß die Echographieaussendung und die Leistungsaussendung den gleichen Anregungsgenerator (10) verwenden, die bei derselben Frequenz arbeiten.

5. Vorrichtung nach Anspruch 1, gekennzeichnet durch eine lineare Schwenkbewegung des Wandlers.

## Claims

1. An ultrasonic treatment device comprising a power transducer in the form of a self-focussing cup (1) associated with a power excitation generator (10) and with an echographic device (13) using said transducer as echo receiver, characterized by means (2) for causing said transducer to oscillate periodically during its use, around a given position at an oscillation speed and amplitude adapted for obtaining real time echographic scanning within a given firing space, and by means (5 to 12) for detecting the position of the transducer and for exciting said transducer with treatment waves at moments when the transducer oscillates in a predetermined restricted region of its oscillating sphere and for exciting it with echographic waves during the intervals between said moments.

2. The device as claimed in claim 1, characterized in that the scanning is sectorial type B scanning and said moments correspond to the passage of the beam in a predetermined restricted angular sector.

3. The device as claimed in claim 2, characterized in that said restricted sector is defined by an angular position sensor (5), whereas the moments of transmission of the treatment wave are defined, from the indications of said sensor, by logic means (6 to 11) generating power transmission square waves.

4. The device as claimed in claim 3, characterized in that the echographic transmission and the power transmission use the same excitation generator (10) working at the same frequency.

5. The device as claimed in claim 1, characterized in that the transducer undergoes a linear oscillating movement.
